# EUROPEAN PATENT SPECIFICATION

(11) **EP 2 943 172 B1**
(45) Date of publication and mention of the grant of the patent: **11.09.2024**
(21) Application number: 14732282.0
(22) Date of filing: 09.01.2014
(51) Int. Cl.: A61F 13/02, A61F 13/42, G01N 27/04, A61F 13/00

(54) **MOISTURE INDICATOR DRESSING**
VERBAND MIT FEUCHTIGKEITSINDIKATOR
PANSEMENT À INDICATEUR D'HUMIDITÉ

(30) Priority: 11.01.2013 GB 201300470
(43) Date of publication of application: 18.11.2015
(73) Proprietor: Smith & Nephew PLC, Watford Hertfordshire WD18 8YE (GB)
(72) Inventor: DAGGER, Anthony, York Yorkshire YO10 5DF (GB); FRY, Nicholas, Charlton, York Yorkshire YO10 5DF (GB); HAMMOND, Victoria, Jody, York Yorkshire YO10 5DF (GB); HICKS, John, Kenneth, York Yorkshire YO10 5DF (GB); LAURIE, Alexander, Speirs, Royston Hertfordshire SG8 6AJ (GB); LECOMTE, Helene, Anne, York Yorkshire YO10 5DF (GB); MOSS, Rhianna, York Yorkshire YO10 5DF (GB); MUMBY, Ella, Lynne, York Yorkshire YO10 5DF (GB)
(74) Representative: Smith & Nephew
(86) International application number: PCT/GB2014/050048
(87) International publication number: WO 2014/108682

(56) References cited:
- WO-A1-02/47737
- WO-A1-2012/074509
- WO-A2-99/12581

## Description

### Background

The field of wound care management has long understood that keeping wounds optimally moist can help the cells in the wound area grow and migrate to the proper location to help the wound heal. Achieving an optimal moist environment relies on good clinical judgement to determine the correct moisture levels, since too little moisture can desiccate the wound and too much can lead to maceration of the wound bed and surrounding tissue. It is therefore important to be able to monitor moisture, to properly and optimally change the bandage, while still allowing the wound heal undisturbed. Common techniques for performing such monitoring rely on visual indications of excess moisture or strikethrough on wound dressings, commonly used indications include visual changes on backing materials or leakage from the dressing.

Some current wound dressings utilize an indicator layer containing a dye which changes colour on contact with wound exudate. A typical dye is gentian violet, which changes from violet to purple when wet, indicating that the dressing is saturated. That change is typically hard to perceive and therefore users often find it unreliable. Other current wound dressing, for example the DuoDerm^{®} Signal dressing sold by Convatec, rely on fluid leaking from the wound into an area behind an impermeable outer covering of the dressing, causing a blister to become visible. Once the edge of the blister reaches an indicator line marked on the outer surface of the dressing changing is required. As the indicator is merely a blister on the surface of the dressing, it is often difficult to read. Additionally, the blister can enlarge and fill with fluid excessive to the requirements of a healing environment and foster an environment for bacterial colonization. There is a need in the art for a moisture indicating wound dressing in which the moisture indicator within the dressing provides a more ascertainable signal to the user. Further, there is a need in the art for a dressing in which the user is able to monitor the moisture levels within different parts of the dressing. Negative pressure wound therapy (NPWT) is a therapeutic technique that utilizes a vacuum dressing to promote wound healing, particularly in chronic wounds. The continued vacuum draws out fluid from the wound and increases blood flow to the area. There is a need to be able to determine within a NPWT system if any part of the fluid outlet tube which is concealed within the dressing has a leak, as this would result in sub-optimal therapy. WO 99/12581 A2 discloses a wound dressing.

### Summary

This application discloses devices related to wound dressings, having moisture indicators. The mechanism of monitoring the moisture levels within the wound dressing is dependent on the moisture indicator becoming exposed or concealed to the user by a physical transformation of a part of the dressing. This provides a visual indication of the dressing's saturation and enables an assessment to be made as to whether the dressing requires changing. Using a moisture indicator within the dressing allows the status of the dressing to be assessed without disturbing the wound and disrupting the healing process.

The invention is defined by the appended claims, which relate to a wound dressing. Embodiments or examples falling outside the scope of the appended claims are provided for understanding only. A wound dressing according to the invention is defined by claim 1. In the preferred embodiment according to the invention, a wound dressing includes an absorbent layer and a moisture indicator which indicates wound exudate loading within the dressing, with the visibility of the moisture indicator changing as a result of a physical transformation of a first material within the dressing. The physical transformation of the first material of the dressing occurs when it is directly contacted by wound exudate. The physical transformation is a change in the appearance of the first material which causes at least a part of the moisture indicator to become revealed or concealed to the user. Such an alteration in the visibility of the moisture indicator provides an indication of the level of saturation. In certain embodiments, the physical transformation is, for example, a transformation from a dry material to a wet material, from a solid material to a gel or gel-like material and *vice versa,* or from a substantially transparent/translucent material to substantially opaque material and *vice versa.* In certain embodiments the transformation is reversible. In some embodiments a plurality of moisture indicators are distributed within the dressing, each indicator being configured to indicate wound exudate loading within a part of the dressing. In certain embodiments, each of the plurality of indicators is a coloured moisture indicator having a different colour, with the visibility of each colour indicating the saturation of a different part of the dressing. In some embodiments, the coloured moisture indicators can be used to indicate the vertical and/or horizontal spread of wound exudate throughout the dressing.

Further areas of applicability of the disclosed devices and methods will become apparent from the detailed description provided hereinafter. It should be understood that the detailed description and specific examples, while indicating particular embodiments, are intended for purposes of illustration only and are not intended to limit the scope of the disclosure or any of the claims that may be pursued.

### Description of the drawings

The foregoing and other objects and advantages will be appreciated more fully from the following further description thereof, with reference to the accompanying drawings. These depicted embodiments are to be understood as illustrative and not limiting in any way:
Figures 1A and 1B are side cross-sectional views of an illustrative wound dressing having a moisture indicator, the visibility of which alters as a result of a physical transformation of a first material of the dressing.
Figures 2A and 2B are side cross-sectional and plan views of an illustrative wound dressing in which a water-soluble coloured moisture indicator becomes visible as the dressing becomes saturated.
Figures 3A and 3B are side cross-sectional and plan views of an illustrative wound dressing in which a coloured moisture indicator becomes invisible as a result of the physical transformation of a first material from transparent to opaque.
Figures 4A and 4B are side cross-sectional and plan views of an illustrative wound dressing in which a coloured moisture indicator becomes visible as a result of the physical transformation of a first material from opaque to transparent.
Figure 5 is a plan view of an illustrative wound dressing in which a coloured moisture indicator is associated with multiple layers of the dressing and becomes visible as a result of the physical transformation of a first material associated with each layer.

### Detailed Description

To provide an understanding of the devices and methods describe herein, certain illustrative embodiments will now be described. For the purpose of clarity and illustration, the wound dressings herein are described as having a coloured moisture indicator. However, other moisture indicators that are non-colour based can be used. Such other additions and modifications will not depart from the scope hereof.

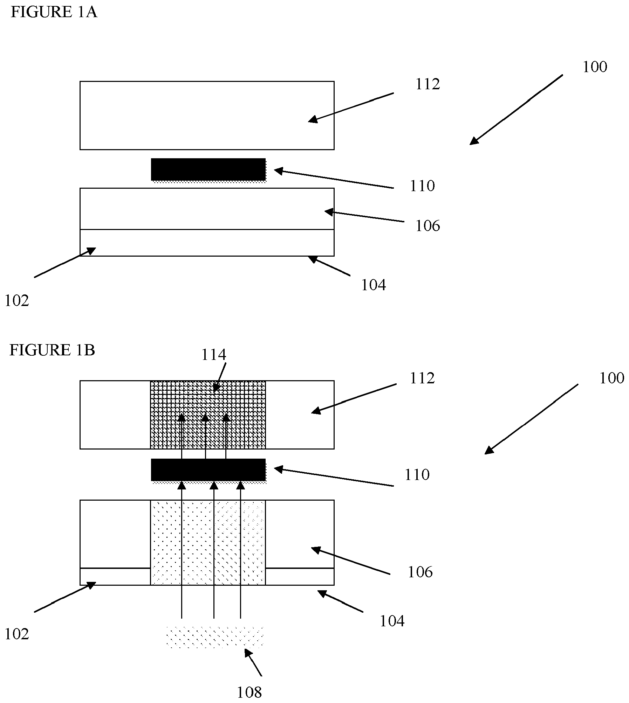

Figures 1A and 1B depict a wound dressing 100 having a wound contacting layer 102 with a wound-facing or wound-contacting surface 104. Figure 1B depicts the wound dressing *in situ* on a wound.

The dressing further includes an absorbent element 106, which can be any material that provides the desired level of absorption of the wound exudate 108. For example, the absorbent element 106 can be a porous foam, particularly a polyurethane foam. Alternatively, the absorbent element 106 can be hydrocolloid-based, hydrogel-based, or alginate-based, or any other suitable absorbent material, or any combination thereof.

The dressing comprises a moisture indicator 110. The moisture indicator is a coloured moisture indicator. The indicator does not necessarily change colour upon contact with wound exudate, but its visibility to the user is altered so that it becomes either visible or invisible. This contrasts with certain moisture indicators found in the existing wound dressing which rely on a colour-change of the indicator itself which is often imperceptible. The coloured moisture indicators of the present application can be advantageous to the user as they provide a discernable visual indication of the moisture levels within the dressing. The colour of the indicator is preferably selected so that it is unambiguously discernable against the other parts of the dressing.

The coloured moisture indicator can be a porous coloured substrate, for example a piece of coloured paper through which the wound exudate can diffuse. Alternatively, the coloured moisture indicator can be a water-soluble coloured dye which, upon solubilisation by the wound exudate, diffuses through the dressing.

The visibility of the moisture indicator 110 is altered as a result of the physical transformation of a first material 112. The first material acts as a mask, which either conceals or exposes the moisture indicator, based on the type of physical transformation. In certain embodiments the physical transformation affects a change in the appearance of the first material, preferably without changing the composition of the indicator. In certain embodiments the physical transformation is a transformation from a dry material to a wet material, from a solid material to a gel or gel-like material and *vice versa,* or from a substantially transparent or translucent material to a substantially opaque material and *vice versa* or a combination thereof. The physical transformation produces a transformed region 114 within the first material 112 and it is this region which affects the visibility of the moisture indicator. In some embodiments, the transformed region 114 functions as a mask which conceals the moisture indicator before the region is physically transformed. Once the region is transformed the mask is disrupted and the moisture indicator becomes visible. In alternative embodiments, the transformed region 114 functions as a mask which conceals the moisture indicator after the region is physically transformed. The transformation of the region results in the formation of the mask and the moisture indicator becomes invisible.

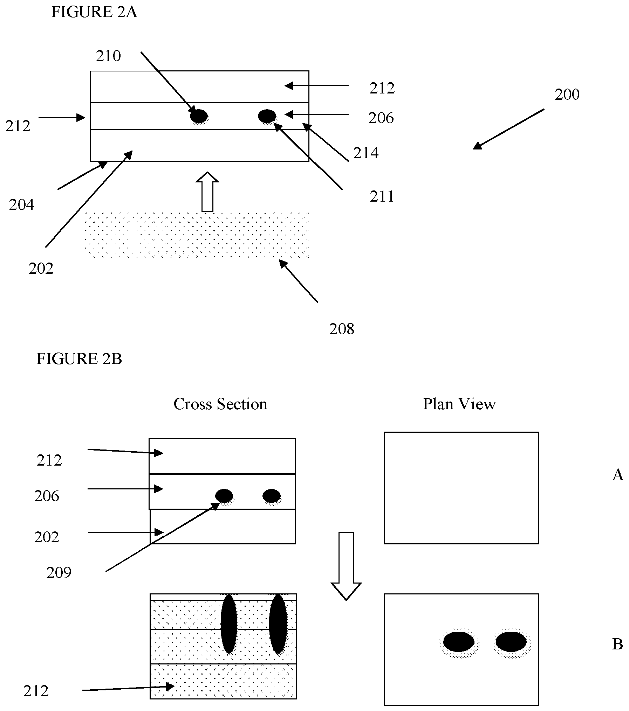

As shown in Figures 2A and 2B, the wound dressing comprises a water-soluble coloured moisture indicator for indicating the saturation levels of the dressing. Figure 2A shows a side cross-sectional view of a wound dressing 200 having a wound contacting layer 202, the lower surface of which is a wound-facing surface 204. The dressing also comprises an absorbent element 206. A first material 212 is located on or adjacent to the surface of the absorbent element which is opposed to the wound-facing surface 204. The first material 212 is of a first colour, preferably white, although it is envisaged that other colours could be utilized. A water-soluble coloured moisture indicator 210, having a second colour, is provided on or within the absorbent layer 206. This second colour is selected to be a contrasting colour to the first colour. For example, if the first colour is white, the second colour is selected to be not white. Suitable water-soluble coloured moisture indicators, for example water-soluble dyes, for use in wound dressings are readily apparent to persons skilled in the art. The absorbent layer has opposing peripheral edges 214 and 216 and although Figures 2A and 2B depict the water-soluble coloured moisture indicator 210 as a mark which is centrally located in the absorbent layer, it is envisaged that the indicator 210 can be located anywhere between the opposing peripheral edges and, if desired, additional indicators (of the same or of a different colour) can be provided at different locations between the peripheral edges in order to provide an indication of the horizontal spread of wound exudate within the absorbent layer. For example, as illustrated, an indicator 211 based on a water-soluble dye of the same colour as indicator 210 is provided nearer to an outer peripheral edge.

Figure 2B, illustrates two stages of wound dressing saturation and the temporal relationship between the solubilisation and subsequent visualisation of the marks 210 and 211. In the upper panels (A) the wound dressing is dry and, from the plan view, it can be seen that the first material is substantially white. This informs the user, for example, the patient or care provider, that the absorbent layer has not been saturated and that the wound dressing does not require changing. As illustrated in the lower panels (B), when wound exudate 208 has saturated the absorbent layer 206, the indicators 210 and 211 become solubilised and diffuse through the first material 212 and become visible as a contrasting coloured mark on the upper surface of the first material. This indicates to the user that the dressing needs to be changed. In some embodiments, the first material 212 forms the uppermost surface of the wound dressing, which is often referred to as the backing layer. In alternative embodiments, the first material 212 is covered by additional layers of the dressing, although such layers are preferably transparent, such that the first material is visible therethrough and the appearance of the coloured mark(s) on the first material is not hampered.

As described above, in some embodiments the coloured moisture indicator appears on the upper surface of the first material, this surface being white or other light colour. The coloured moisture indicator is of a contrasting colour to white, so that the apparent "white to colour" transition provides an unambiguous visual indication as to dressing saturation.

Methods for fabricating wound dressing products are also contemplated. An example includes the following steps for making the dressing illustrated in Figure 2, as outlined below:

In a first step, a first coating (filler layer) is applied to the upper side of a sheet of conventional copy paper (~80GSM). Coating 1 (~100 GSM) may be a dispersion of talc, or other transparent filler, in a water-soluble binder such as carboxymethylcellulose. The dispersion is preferably formulated with a high pigment/binder ratio to provide satisfactory porosity. The function of this coating is to mask traces of the underlying coloured moisture indicator.

In a second step, the lower side of the paper is coated with a second coating (barrier layer). An example barrier layer is coating 2 (-5-10 GSM), a thin film of polyvinyl alcohol (PVOH). The function of this coating is to seal the paper, in order to impair or prevent the low viscosity ink from diffusing through the paper upon its application.

In a third step, ink is applied to the surface of the second coating, by inkjet or similar deposition method. An example ink is a low-viscosity ink, based on a water-soluble dye.

The response time of the indicator is typically about 10 minutes, but the response time may vary as a function of the paper porosity, thickness of the barrier film and/or the solubility of the dye. In certain applications although the coated paper may react with liquid water, it is not affected by the high levels of humidity associated with sterilization of the wound dressing (e.g by ethylene oxide).

Methods of fabricating alternative specific embodiments of the structure illustrated in Figure 2 include;
In a first step, the filler layer is applied to the lower surface of the paper so that it is sandwiched between the paper and the barrier layer.

In a second step, a dispersion of aluminium hydroxide is used to form the filler layer. This compound may cause the mobile ink to instantly gel, so preventing if from diffusing before it is contacted by wound exudate.

In a third step, titanium dioxide is added to the filler dispersion to emphasise the contrast of colour against a white background without significantly reducing the colour strength.

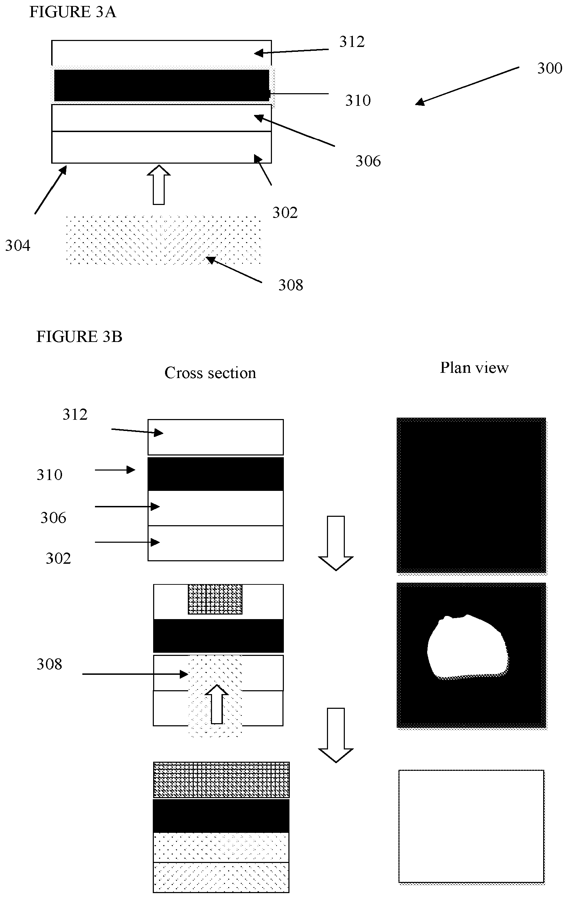

In certain embodiments, the wound dressing comprises a coloured moisture indicator which gradually becomes invisible as the wound dressing becomes saturated. Such a mechanism is shown in Figures 3A and 3B.

Figure 3A shows a side cross-sectional view of a wound dressing 300 having a wound contacting layer 302, the lower surface of which is a wound-facing surface 304. The dressing also comprises an absorbent element 306. Located on the surface of the absorbent element which is opposed to the wound-facing surface 304 is a first material 312 which is made of a material which, upon hydration, transforms from a transparent material to an opaque material having a first colour. Many polymers, such as solutions of polyvinyl acetate (PVA), shellac and latex rubber form transparent films when the carrier solvent evaporates. When the film subsequently comes into contact with water, the polymer re-hydrates to form a white, opaque film. When PVA is bought into contact with water, a relatively high level of opacity develops within a short length of time. This can provide a real-time indication of wound dressing saturation. As illustrated, a coloured moisture indicator 310 having a second colour lies between the absorbent layer and the first material. The second colour is selected to contrast with the colour of the opaque film. For example, if a PVA film is used, which forms a white opaque film upon hydration, the second colour is selected to be non-white and is desirably selected to be a colour that readily contrasts with white. Whilst the material selected for the first material is preferably able to physically transform from a transparent material to an opaque material, it is not a requirement that the first material is colourless i.e clear.

In some embodiments, the coloured moisture indicator 310 is in close contact with, for example, overlies the absorbent layer. In some embodiments the coloured moisture indicator 310 contacts only part of the absorbent layer 306, for example, the central part or a peripheral part. For example, the coloured moisture indicator 310 may form a peripheral edge upon the upper surface of the absorbent layer 306, and the subsequent visibility of the coloured indicator indicates full saturation of the dressing to the peripheral edges. In certain embodiments the coloured indicator is a porous coloured substrate, for example a coloured paper. In other embodiments a coloured mark/graphic (e.g a logo) is applied directly onto the upper surface of the absorbent layer. In some embodiments of the invention the first material 312 forms the uppermost surface of the wound dressing, which is often referred to as the backing layer. In alternative embodiments, the first material 312 is covered by additional layers of the dressing, although such layers are transparent, such that the first material is visible there through. In certain embodiments the physical transformation is reversible. For example, upon hydration the PVA film forms a white opaque film, but if the wound starts to dry out, which is indicative of suboptimal healing, the opaque film reverts to a transparent film and the coloured moisture indicator becomes visible again.

Figure 3B illustrates two stages of wound dressing saturation and the temporal relationship between the wound dressing saturation and the visibility of the coloured moisture indicator 310. In the upper panels (A) the wound dressing is dry and from the plan view of the dressing it can be seen that the first material 312 is completely transparent and the coloured moisture indicator 310 is visible through the first material 310. This informs the user, for example the patient or care provider, that the absorbent layer has not been saturated and that the wound dressing does not require changing. As illustrated in the lower panels (B), when the wound exudate saturates the absorbent layer 306, it permeates through the coloured moisture indicator 310 and contacts the first material 312. This contact hydrates the first material 312 and causes the development of an emulsion, turning the transparent first material 312 opaque. The opaqueness conceals the coloured moisture indicator 310, for example a coloured substrate or a coloured mark/graphic (e.g a logo) which is associated with the absorbent layer. When a predetermined amount of the coloured moisture indicator has become concealed, it indicates that it is time to change the dressing. A reference guide can be provided which informs the user of the relationship between the visibility of the coloured moisture indicator and the level of saturation of the dressing, in order that an informed decision about the need to change the dressing can be taken.

In certain embodiments, the wound dressing is a superabsorbent dressing utilized in a Negative Pressure Wound Therapy (NPWT) System, for example the dressing of the PICO^{®} Single Use Negative Pressure Wound Therapy System (Smith & Nephew Inc.). Such dressings are provided with a port into which a vacuum tube is secured. It could be advantageous for the coloured moisture indicator to be associated with this port, with the concealment of the coloured moisture indicator being indicative of a fully saturated dressing, which should be removed.

As described above, in some embodiments the coloured moisture indicator becomes concealed by the development of an opaque film. In embodiments in which the opaque film is white in colour, the coloured moisture indicator may be a contrasting colour to white, so the apparent "colour to white" transition provides an unambiguous visual indication as to dressing saturation.

An Example method for fabricating a specific embodiment of the structure illustrated in Figure 3 is outlined below:
In a first step, a film of PVA emulsion is deposited onto a thin (e.g 35 micron) a polyester/plastic film, for example Mylar^{®} (Dupont). This prevents the reticulation of the converted PVA. The PVA is preferably a ~50% aqueous emulsion, for example Unibond (Henkle) or Cementone Rendabond (Bostik).
In a second step, the wet film is oven-dried (e.g to about 70 degrees Celsius) to provide a transparent PVA film of about 100 microns thick. The PVA/Mylar laminate is then bonded to an appropriately coloured material (e.g a, 2- ply paper tissue (~35 GSM)) using an adhesive such as polyvinyl alcohol or K5 adhesive (Smith & Nephew, Inc).
In a third step, the laminate prepared in step 2 is adhered to the upper surface of the absorbent layer in a wound dressing.

Although PVA film may react with liquid water, it is not necessarily affected by the high levels of humidity associated with the ethylene oxide sterilization of the wound dressing.

Methods of fabricating alternative embodiments of the structure illustrated in Figure 3 include:
In a first step, the PVA/Mylar layer is bonded directly onto an appropriate coloured piece of fusible interlining, for example based on non-woven rayon.
In a second step, the Mylar film is omitted and instead the layer of PVA emulsion is screen printed, or otherwise directly deposited, onto the porous coloured substrate.
In a third step, the porous coloured tissue paper is omitted and the coloured mark/graphic (e.g a logo) is directly applied onto the upper surface of the absorbent layer of the dressing.
In a fourth step, the use of an adhesive is omitted by instead hot bonding the PVA/Mylar laminate onto the porous coloured substrate.
In a fifth step, the porous coloured substrate is omitted and instead a pattern (e.g a chequerboard) or other graphic element (e.g a logo) is directly applied onto the uppermost transparent film (e.g PVA/Mylar laminate) such that the pattern can be displayed as a more distinctive change signal.

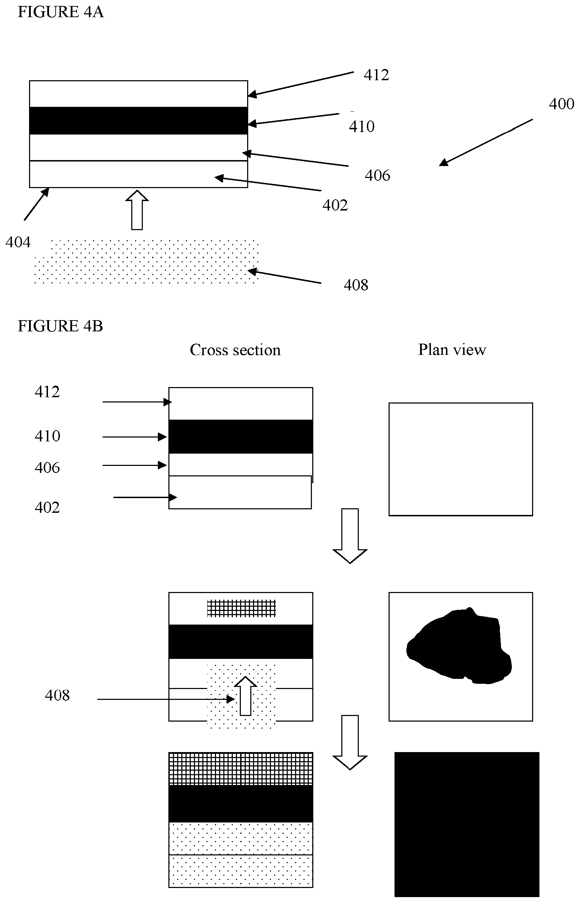

In certain embodiments the wound dressing comprises a coloured moisture indicator which gradually becomes visible as the wound dressing becomes saturated. Such a mechanism is shown in Figure 4A and 4B.

Figure 4A shows a side cross-sectional view of a wound dressing 400 having a wound contacting layer 402, the lower surface of which is the wound-facing surface 404. The dressing also comprises an absorbent element 406. Located on the surface of the absorbent element which is opposed to the wound-facing surface 404 is a first material 412, made of a material which upon hydration transforms from being opaque to a transparent/translucent. A suitable material is a water-soluble paper, which is preferably a polyvinyl alcohol-based. The water-soluble paper has a fibrous structure which upon hydration transforms and disintegrates into a transparent/translucent gel or gel-like material. There are many types of suitable water soluble papers available, including, for example, water-soluble papers based on plant materials such as rice, cornflour and cellulose or based on synthetic polymers, such as polyvinyl alcohol, the base for the Aquasol (Aquasol Corp) range. The water-soluble paper is preferably white. As illustrated, a coloured moisture indicator 410 having a second colour lies between the absorbent layer and the first material. The second colour is selected to contrast with the colour of the opaque water-soluble paper. For example, if the non-hydrated form of the water-soluble paper is white, then the second colour is selected to be non-white and is preferably a colour that readily contrasts with white. In some embodiments, the coloured moisture indicator 410 is in close contact with the absorbent layer, for example, it may overly the absorbent layer. In some embodiments, the coloured moisture indicator 410 contacts only part of the absorbent layer, for example, the central part or a peripheral part. The coloured moisture indicator 410 may form a peripheral edge upon the upper surface of the absorbent layer, such that the subsequent visibility of the coloured indicator indicates of full saturation of the dressing to the peripheral edges. When the practitioner sees that the indication he or she may decide to change the dressing. In certain embodiments the coloured indicator is a porous coloured substrate, for example a coloured paper. In other embodiments a coloured mark/graphic (e.g a logo) is applied directly onto the upper surface of the absorbent layer. In some embodiments, the first material 412 forms the uppermost surface of the wound dressing, which is often referred to as the backing layer. In alternative embodiments, the first material 412 is covered by additional layers of the dressing, although such layers are transparent, such that the first material is visible there through. In some embodiments, an additional coloured element is disposed between the first material 412 and the coloured moisture indicator 410. This additional coloured element has a third colour, which is of a contrasting colour to the second colour. In this embodiment, the first material 412 and the additional coloured element do not necessarily extend completely over the coloured moisture indicator 410, such that when the first material 412 becomes hydrated and dissolves, the third colour becomes visible against the coloured moisture indicator. In certain embodiments, the wound dressing is a superabsorbent dressing utilized in a Negative Pressure Wound Therapy (NPWT) System, for example the dressing of the PICO^{®} Single Use Negative Pressure Wound Therapy System (Smith & Nephew Inc, ). Such dressings are provided with a port into which a vacuum tube is attached to. If the coloured moisture indicator is associated with this port, the exposure of the coloured moisture indicator may indicate a fully saturated dressing, which should be removed.

Figure 4B illustrates two stages of wound dressing saturation and illustrates the temporal relationship between the wound dressing saturation and the visibility of a coloured moisture indicator 408. In the upper panels (A) the wound dressing is dry and from the plan view of the dressing it can be seen that the first material 412 is completely opaque and the coloured moisture indicator 410 is invisible through the backing layer. This informs the user, for example the patient or care provider, that the absorbent layer has not been saturated and that the wound dressing does not require changing. As illustrated in the lower panels (B), when the wound exudate 408 has saturated the absorbent layer 406, it permeates through the coloured substrate 410 and contacts the first material 412. This contact hydrates the first material 412, causing the development of a transparent/translucent gel. This transparency exposes the coloured moisture indicator 410 beneath. When a predetermined amount of the second colour has become exposed it is time to change the dressing. A reference guide can be provided which informs the user of the relationship between the visibility of the coloured moisture indicator and the level of saturation of the dressing, in order that an informed decision about the need to change the dressing can be made.

As described above, in some embodiments the coloured moisture indicator becomes exposed by the development of a transparent/translucent film. The coloured moisture indicator may be a contrasting colour to white, so the apparent "white to colour" transition, provides an unambiguous visual indication as to dressing saturation.

An Example method for fabricating an embodiment of the structure illustrated in Figure 4 is outlined below:
In a first step, a water-soluble paper (~60GSM) is bonded to an appropriately coloured material (e.g. a 2-ply, paper tissue (~35 GSM)) using an adhesive such as polyvinyl alcohol or K5 adhesive (Smith & Nephew, Inc), to form a laminate.
In a second step, the laminate prepared in Step 1 is adhered to the upper surface of an absorbent layer in a wound dressing.

Although the laminate reacts with liquid water, it not necessarily affected by the high levels of humidity associated with sterilization of the wound dressing (e.g by ethylene oxide).

Methods of fabricating alternative embodiments of the structure illustrated in Figure 4 include:
In a first step, the water-soluble paper is bonded directly onto an appropriate coloured piece of fusible interlining, for example based on non-woven rayon.
In a second step, the use of an adhesive is omitted and instead an adherent water-soluble paper is used.

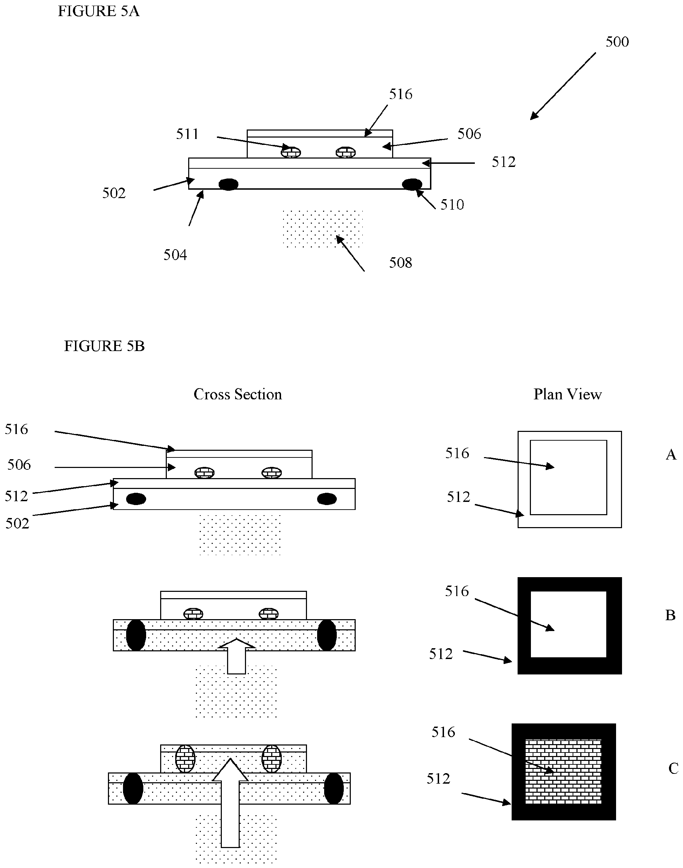

In certain embodiments, the wound dressing includes a coloured moisture indicator associated with each layer of the dressing, such that the user is visually informed of the vertical progression of the wound exudate and therefore the saturation state of each dressing layers. An example of this design of dressing is illustrated in Figures 5A and 5B in which the wound dressing comprises a water-soluble coloured moisture indicator associated with each layer of the dressing.

Figure 5A shows a side cross-sectional view of a wound dressing 500 having a wound contacting layer 502, the lower surface of which is a wound-facing surface 504. A first material 512 is located on or adjacent to the surface of the wound contacting layer 502 which is opposed to the wound-facing surface 204. The wound contacting layer 502 and the first material 512 are preferably of the same diameter. The dressing also comprises an absorbent element 506. A second material 516 is located on or adjacent to the surface of the absorbent element 506 which is opposed to the wound-facing surface 504. The absorbent element 506 and the second material 516 are preferably of the same diameter, with this diameter being selected to be smaller than the diameter of the wound contacting layer 502 and the first material 512. As a result of this selection, a border or flange of the wound contacting layer 502/first material 512 extends outwardly from below the perimeter edges of the absorbent element 506/second material 516. The first material 512 and the second material 516 are of a first colour, preferably white, although it is envisaged that other colours could be utilized. A water-soluble coloured moisture indicator 510, having a second colour, is provided on or within the wound contacting layer 502. A water-soluble coloured moisture indicator 511, having a third colour, is provided on or within the absorbent layer 506. The second and third colours are selected to be a contrasting colour to the first colour. For example, if the first colour is white, the second and third colours are selected to be not white. Suitable water-soluble coloured moisture indicators, for example water-soluble dyes, for use in wound dressings are readily apparent to persons skilled in the art.

Figure 5B, illustrates two stages of wound dressing saturation and the temporal relationship between the solubilisation and subsequent visualisation of the marks 510 and 511. In the upper panels (A) the wound dressing is dry and, from the plan view, it can be seen that the first material and the second material are substantially white. This informs the user, for example, the patient or care provider, that neither the wound contacting layer, nor the absorbent layer, has been saturated by wound exudate. As illustrated in the middle panels (B), when wound exudate 508 has saturated the wound contacting layer 502 the indicator 510 is solubilised and diffuses through the first material 512 and becomes visible as a contrasting coloured mark on the upper surface of the first material 512. In some embodiments, the first material 512 forms the uppermost surface of the wound contacting layer. The visibility of the indicator 510 indicates to the user that the wound contacting layer 502 has become saturated. This may not necessarily indicate that a dressing change is required, but it may be a useful indication to the user of the rate of wound exudate secretion from the wound. As illustrated in the lower panels (C), when wound exudate 508 has saturated the absorbent layer 506, the indicator 511 is solubilised and diffuses through the second material 516 to become visible as a contrasting coloured mark on the upper surface of the second material 516. In some embodiments, the second material 516 forms the uppermost surface of the wound contacting layer. In alternative embodiments, the second material 516 is covered by additional layers of the dressing, although such layers are preferably transparent, such that the second material is visible there through and the appearance of the coloured mark(s) on the second material is not hampered. The visibility of the indicator 511 indicates to the user that the absorbent layer of the dressing has become saturated and that the dressing may require changing.

As described in relation to Figures 5A and 5B, in certain embodiments, the wound dressing includes a coloured moisture indicator associated with each layer of the dressing, this enables the user to be visually informed as each layer of the dressing becomes saturated by the vertical progression of the wound exudate. Whilst Figures 5A and 5B disclose the use of water-soluble dyes as the moisture indicator, in alternative embodiments, it is envisaged that the moisture indicators disclosed in reference to Figures 3 and 4 may be utilized within a similar design of dressing, as described below.

In embodiments, the first and second materials 512, 516 that are associated with the wound contacting layer 502 and the absorbent layer 506, respectively, are made of a material which, upon hydration, transforms from a transparent material to an opaque material having a first colour, as disclosed above in reference to Figure 3. The first colour is preferably white. A first coloured moisture indicator, for example a coloured substrate or a coloured mark/graphic (e.g a logo), is placed between the wound contacting layer and the first material. The first coloured moisture indicator is of a colour that is contrasting to white. A second coloured moisture indicator, for example a coloured substrate or a coloured mark/graphic (e.g a logo), is placed between the absorbent layer and the second material. The second coloured moisture indicator is also of a colour that is contrasting to white and further is discernable from the colour of the first coloured moisture indicator. Within the dry dressing, both the first and second coloured moisture indicators are visible to the user. When the wound contacting layer becomes saturated the first material becomes hydrated and turns opaque, concealing the first coloured moisture indicator. When the absorbent layer becomes saturated the second material becomes hydrated and turns opaque, concealing the second coloured moisture indicator. The fully hydrated dressing will appear white to the user.

In embodiments, the first and second materials 512, 516 that are associated with the wound contacting layer 502 and the absorbent layer 506, respectively, are made of a material which, upon hydration, transforms from an opaque material having a first colour to a transparent material, as disclosed above in reference to Figure 4. The first colour is preferably white. A first coloured moisture indicator, for example a coloured substrate or a coloured mark/graphic (e.g a logo), is placed between the wound contacting layer and the first material. The first coloured moisture indicator is of a colour that is contrasting to white. A second coloured moisture indicator, for example a coloured substrate or a coloured mark/graphic (e.g a logo), is placed between the absorbent layer and the second material. The second coloured moisture indicator is also of a colour that is contrasting to white and further is discernable from the colour of the first coloured moisture indicator. Within the dry dressing, both the first and second coloured moisture indicators are invisible to the user. When the wound contacting layer becomes saturated the first material becomes hydrated and turns transparent, exposing the first coloured moisture indicator. When the absorbent layer becomes saturated the second material becomes hydrated and turns transparent, exposing the second coloured moisture indicator. The fully hydrated dressing will have a central first colour and a ring of a second colour extending thereabouts.

It is to be understood that the foregoing description is merely illustrative and is not to be limited to the details given. While several embodiments have been provided in the present disclosure, it should be understood that the disclosed devices and method and their components, may be embodied in many other specific forms without departing from the scope of the disclosure.

Variations and modifications will occur to those of skill in the art after reviewing this disclosure. The disclosed features may be implemented, in any combination and sub-combinations (including multiple dependent combinations and sub-combinations), with one or more other features described herein. The various features described or illustrated above, including any components thereof, may be combined or integrated in other systems. Moreover, certain features may be omitted or not implemented.

## Claims

1. A wound dressing for use in a Negative Pressure Wound Therapy (NPWT) System (100, 300, 400) comprising:
a wound contacting layer (102, 302, 402) the lower surface of which is a wound-facing surface (104, 304, 404);
an absorbent layer (106, 306, 406) and
a first material (112, 312, 412) located on the absorbent layer (106, 306, 406) which is opposed to the wound-facing surface (104, 304, 404),
a moisture indicator which indicates wound exudate loading within the dressing, wherein the moisture indicator is positioned between the absorbent layer and the first material, wherein the visibility of the moisture indicator changes as a result of hydration of the first material (112, 312, 412) within the dressing when it is directly contacted by wound exudate,
wherein either
hydration of the first material when it is directly contacted by wound exudate transforms its appearance from substantially opaque having a first colour to substantially transparent or translucent, thereby causing at least part of the moisture indicator to become visible;
or
hydration of the first material when it is directly contacted by wound exudate transforms its appearance from being substantially transparent or translucent to substantially opaque having a first colour, thereby causing at least part of the moisture indicator to become invisible,
wherein the moisture indicator is a coloured moisture indicator having a second colour, wherein the second colour is selected to be a contrasting colour to the first colour.

2. A wound dressing according to claim 1, wherein the wound dressing is a superabsorbent dressing.

3. A wound dressing according to claim 1, wherein the first material comprises a water soluble material which when dry is substantially opaque in appearance and when wet is hydrated and physically transforms into a gel which is substantially transparent or translucent in appearance.

4. A wound dressing according to claim 1, wherein the first material comprises a film which when dry is substantially transparent or translucent in appearance and when wet is hydrated and forms an emulsion which is substantially opaque in appearance.

5. A wound dressing according to claim 4, wherein the film is polyvinyl acetate.

6. A wound dressing according to claim 1, wherein the first material that undergoes a transformation in its appearance is substantially white, either pre- or post- transformation and the colour of the moisture indicator is selected not to be substantially white.

## Patentansprüche

1. Ein Wundverband zur Verwendung in einem Unterdruckwundtherapie-System, NPWT-System, (100, 300, 400), beinhaltend:
eine Wundkontaktschicht (102, 302, 402), deren untere Fläche eine der Wunde zugewandte Fläche (104, 304, 404) ist;
eine absorbierende Schicht (106, 306, 406) und
ein erstes Material (112, 312, 412), das sich auf der absorbierenden Schicht (106, 306, 406) befindet, die der der Wunde zugewandten Fläche (104, 304, 404) gegenüberliegt, einen Feuchtigkeitsindikator, der Wundexsudatbelastung innerhalb des Verbandes anzeigt, wobei der Feuchtigkeitsindikator zwischen der absorbierenden Schicht und
dem ersten Material positioniert ist, wobei sich die Sichtbarkeit des Feuchtigkeitsindikators als Folge der Hydratation des ersten Materials (112, 312, 412) innerhalb des Verbandes ändert, wenn es direkt mit Wundexsudat in Kontakt kommt,
wobei entweder
die Hydratation des ersten Materials, wenn es direkt mit Wundexsudat in Kontakt kommt, sein Aussehen von im Wesentlichen undurchsichtig mit einer ersten Farbe in im Wesentlichen durchsichtig oder lichtdurchlässig verwandelt, wodurch bewirkt wird, dass mindestens ein Teil des Feuchtigkeitsindikators sichtbar wird;
oder
die Hydratation des ersten Materials, wenn es direkt mit Wundexsudat in Kontakt kommt, sein Aussehen von im Wesentlichen durchsichtig oder lichtdurchlässig in im Wesentlichen undurchsichtig mit einer ersten Farbe verwandelt, wodurch bewirkt wird, dass mindestens ein Teil des Feuchtigkeitsindikators unsichtbar wird,
wobei der Feuchtigkeitsindikator ein farbiger Feuchtigkeitsindikator mit einer zweiten Farbe ist, wobei die zweite Farbe so gewählt ist, dass sie eine Kontrastfarbe zu der ersten Farbe ist.

2. Wundverband gemäß Anspruch 1, wobei der Wundverband ein superabsorbierender Verband ist.

3. Wundverband gemäß Anspruch 1, wobei das erste Material ein wasserlösliches Material beinhaltet, das im trockenen Zustand ein im Wesentlichen undurchsichtiges Aussehen hat und im feuchten Zustand hydratisiert ist und sich physisch in ein Gel verwandelt, das ein im Wesentlichen durchsichtiges oder lichtdurchlässiges Aussehen hat.

4. Wundverband gemäß Anspruch 1, wobei das erste Material eine Folie beinhaltet, die im trockenen Zustand ein im Wesentlichen durchsichtiges oder lichtdurchlässiges Aussehen hat und im feuchten Zustand hydratisiert ist und eine Emulsion bildet, die ein im Wesentlichen undurchsichtiges Aussehen hat.

5. Wundverband gemäß Anspruch 4, wobei die Folie Polyvinylacetat ist.

6. Wundverband gemäß Anspruch 1, wobei das erste Material, das eine Verwandlung seines Aussehens erfährt, im Wesentlichen weiß ist, entweder vor oder nach der Verwandlung, und die Farbe des Feuchtigkeitsindikators so gewählt ist, dass sie im Wesentlichen nicht weiß ist.

## Revendications

1. Un pansement pour plaie destiné à être utilisé dans un système de Traitement des plaies par Pression Négative (TPN) (100, 300, 400) comprenant :
une couche en contact avec la plaie (102, 302, 402) dont la surface inférieure est une surface faisant face à la plaie (104, 304, 404) ;
une couche absorbante (106, 306, 406) et
un premier matériau (112, 312, 412) situé sur la couche absorbante (106, 306, 406) qui est à l'opposé de la surface faisant face à la plaie (104, 304, 404),
un indicateur d'humidité qui indique une accumulation d'exsudat de plaie au sein du pansement, où l'indicateur d'humidité est positionné entre la couche absorbante et le premier matériau, où la visibilité de l'indicateur d'humidité change en conséquence de l'hydratation du premier matériau (112, 312, 412) au sein du pansement lorsque de l'exsudat de plaie entre directement en contact avec lui,
où soit
l'hydratation du premier matériau lorsque de l'exsudat de plaie entre directement en contact avec lui transforme son aspect de substantiellement opaque présentant une première couleur à substantiellement transparent ou translucide, amenant de ce fait au moins une partie de l'indicateur d'humidité à devenir visible ;
soit
l'hydratation du premier matériau lorsque de l'exsudat de plaie entre directement en contact avec lui transforme son aspect de substantiellement transparent ou translucide à substantiellement opaque présentant une première couleur, amenant de ce fait au moins une partie de l'indicateur d'humidité à devenir invisible,
où l'indicateur d'humidité est un indicateur d'humidité coloré présentant une deuxième couleur, où la deuxième couleur est sélectionnée pour être une couleur qui contraste avec la première couleur.

2. Un pansement pour plaie selon la revendication 1, où le pansement pour plaie est un pansement superabsorbant.

3. Un pansement pour plaie selon la revendication 1, où le premier matériau comprend un matériau soluble dans l'eau qui, lorsqu'il est sec, est d'aspect substantiellement opaque et, lorsqu'il est mouillé, est hydraté et se transforme physiquement en un gel qui est d'aspect substantiellement transparent ou translucide.

4. Un pansement pour plaie selon la revendication 1, où le premier matériau comprend un film qui, lorsqu'il est sec, est d'aspect substantiellement transparent ou translucide et, lorsqu'il est mouillé, est hydraté et forme une émulsion qui est d'aspect substantiellement opaque.

5. Un pansement pour plaie selon la revendication 4, où le film est de l'acétate de polyvinyle.

6. Un pansement pour plaie selon la revendication 1, où le premier matériau qui fait l'objet d'une transformation de son aspect est substantiellement blanc, soit préalablement à, soit postérieurement à la transformation et la couleur de l'indicateur d'humidité est sélectionnée pour ne pas être substantiellement blanche.
